# EUROPÄISCHE PATENTANMELDUNG

(11) **EP 2 386 652 A1**
(43) Veröffentlichungstag der Anmeldung: **16.11.2011**
(21) Anmeldenummer: 10005011.1
(22) Anmeldetag: 12.05.2010
(51) Int. Cl.: C12Q 1/37, C12Q 1/56, G01N 33/68

(54) **Homogenes Verfahren zur Bestimmung der Plasminogenaktivator-aktivierenden Aktivität der FSAP**

(71) Anmelder: Siemens Healthcare Diagnostics Products GmbH, 35041 Marburg (DE)
(72) Erfinder: Schwarz, Herbert, 35102 Lohra (DE); Vitzthum, Frank, Dr., 35094 Lahntal (DE)

(57) **Zusammenfassung**

Die Erfindung liegt auf dem Gebiet der in vitro-Diagnostik und betrifft ein homogenes Verfahren zur Bestimmung der Plasminogenaktivator-aktivierenden Aktivität der den Blutgerinnungsfaktor VII-aktivierenden Protease (FSAP). Durch den Zusatz eines Aktivators und durch eine pH-Wert-Optimierung kann auf Schritte zur Anreicherung der FSAP verzichtet werden.

## Beschreibung

Die Erfindung liegt auf dem Gebiet der in vitro-Diagnostik und betrifft ein homogenes Verfahren zur Bestimmung der Plasminogenaktivator-aktivierenden Aktivität der den Blutgerinnungsfaktor VII-aktivierenden Protease (FSAP).

Die FSAP ist eine plasmatische Serinprotease, die auch unter dem Namen PHBSP (plasma hyaluronan binding serine protease) beschrieben wurde. Die FSAP liegt im Humanplasma in einer Konzentration von ca. 12 µg/ml vor und kann über Autokatalyse vom einkettigen Proenzym (single chain-, sc-FSAP) in die aktive zweikettige Protease (two chain-, tc-FSAP) überführt werden (Etscheid, M. et al.(2000) Activation of proPHBSP, the zymogen of a plasma hyaluronan binding serine protease, by an intermolecular autocatalytic mechanism. Biol. Chem. 381, 1223-1231; Kannemeier, C., et al. (2001) Factor VII and single-chain plasminogen activator-activating protease, activation and autoactivation of the proenzyme. Eur. J. Biochem. 268, 3789-3796). Die aktive Protease verfügt über verschiedene Funktionen bzw. Aktivitäten. Es ist bekannt, dass die FSAP einerseits die Fähigkeit besitzt, den Blutgerinnungsfaktor VII sowie Einketten-Plasminogenaktivatoren, wie Plasminogen-aktivierende Prourokinasen, wie scuPA (single chain urokinase plasminogen activator) und sctPA (single chain tissue plasminogen activator) zu aktivieren. Andererseits verfügt die FSAP über die Fähigkeit, die Blutgerinnungsfaktoren V/Va und VIII/VIIIa zu inaktivieren.

Die Bestimmung einer verminderten FSAP-Aktivität in Patientenproben ermöglicht die Diagnose eines erhöhten Risikos für thrombotische und atherosklerotische Erkrankungen (siehe z. B. EP 1348761 A1). Insbesondere Träger der Marburg I (MRI)-Mutation der FSAP, die bei ungefähr 5 bis 10 % der westeuropäischen Bevölkerung gefunden wird, weisen eine verminderte FSAP-Aktivität und damit ein erhöhtes Thrombose- und Atheroskleroserisiko auf.

Verschiedene Testverfahren zur qualitativen oder quantitativen Bestimmung der FSAP sind in EP 952215 A2 beschrieben. Für die Bestimmung der Plasminogenaktivator-aktivierenden Aktivität der FSAP in Plasmaproben werden bislang ausschließlich heterogene Verfahren eingesetzt. In EP 1059359 A2 ist das heterogene Verfahren beschrieben, das üblicherweise zur Bestimmung der Plasminogenaktivator-aktivierenden Aktivität der FSAP in Plasmaproben verwendet wird. Eine Plasmaprobe wird zunächst mit einem FSAP-spezifischen, Festphasen-gekoppelten Bindungspartner, üblicherweise einem Antikörper, inkubiert, die Festphase wird zur Abtrennung nicht gebundener Probenbestandteile gewaschen, und anschließend wird die Festphasen-gebundene FSAP mit einem Plasminogenaktivator, z. B. Prourokinase, und einem markierten Peptidsubstrat für den aktivierten Plasminogenaktivator inkubiert, und die Spaltung des Peptidsubstrats wird photometrisch bestimmt.

Der Nachteil solcher heterogener Verfahren besteht darin, dass sie mindestens einen Separationsschritt, wenn nicht sogar weitere Waschschritte, umfassen. Diese zusätzlichen Verfahrensschritte erschweren die Automatisierung und verlängern die Bearbeitungszeit. Die Bearbeitungszeit verlängert sich auch dadurch, dass die Probe für einen gewissen Zeitraum mit der Festphase inkubiert werden muss, um die Bindung der FSAP an die Festphase zu ermöglichen. Ein weiterer Nachteil besteht darin, dass durch die Inkubation, Separation und das Waschen der Festphase die Aktivität der FSAP beeinflusst werden kann. Beispielsweise können Konformationsänderungen oder Degradierung zu Aktivitätsänderungen führen. Ein weiterer Nachteil der heterogenen Verfahren besteht darin, dass andere Probenbestandteile abgetrennt oder zumindest deren Konzentrationen reduziert werden, so dass probenintrinsische Bindungspartner, Cofaktoren, Aktivatoren oder Inhibitoren, welche die Aktivität der FSAP beeinflussen, ebenfalls abgetrennt werden, so dass keine Bestimmung der Enzymaktivität im physiologischen Kontext der Probenmatrix möglich ist.

Der vorliegenden Erfindung lag daher die Aufgabe zugrunde, ein homogenes Verfahren bereit zu stellen, das die spezifische Bestimmung der Prourokinase-aktivierenden Aktivität der FSAP in komplexen Proteinlösungen, insbesondere in biologischen Proben, wie Plasmaproben, ermöglicht.

Die Aufgabe wird dadurch gelöst, dass die zu untersuchende Probe vor der Zugabe der Reagenzien, die für den Nachweis der Prourokinase-aktivierenden Aktivität der FSAP benötigt werden, d. h. vor Zugabe des Plasminogenaktivators, mit mindestens einem Aktivator der FSAP bei einem pH-Wert von 4 bis 8 inkubiert wird.

Gegenstand der vorliegenden Erfindung ist daher ein homogenes Verfahren zur Bestimmung der Plasminogenaktivator-aktivierenden Aktivität der Faktor VII-aktivierenden Protease (FSAP) in einer Probe, wobei die Probe mit einem Plasminogenaktivator und einem Plasminogenaktivator-spezifischen Substrat vermischt wird und die Plasminogenaktivatoraktivität gemessen wird, wobei der Probe vor der Zugabe des Plasminogenaktivators ein Aktivator der FSAP-Aktivität zugegeben wird und der pH-Wert der Probe auf einen Wert von 4 bis 8 eingestellt wird.

Unter einem homogenen Verfahren ist ein Verfahren zu verstehen, das ohne Separations- oder Waschschritte auskommt, die der Abtrennung und/oder Anreicherung der FSAP aus der Probe dienen würden.

Der Begriff "Plasminogenaktivator" umfasst Einketten-Plasminogenaktivatoren, wie Plasminogen-aktivierende Prourokinasen, wie scuPA (single chain urokinase plasminogen activator) und sctPA (single chain tissue plasminogen activator).

Zur Bestimmung der Plasminogenaktivator-aktivierenden Aktivität der FSAP wird die Probe mit einem inaktiven Einketten-Plasminogenaktivator, wie z. B. Prourokinase oder sct-PA und einem Urokinase-Substrat oder einem sct-PA-Substrat inkubiert. Die FSAP-abhängige Aktivierung von z. B. Prourokinase zu Urokinase wird anhand der Umsetzung bzw. Spaltung des Urokinase-Substrats gemessen. Bevorzugte Substrate sind niedermolekulare Peptidsubstrate, die über eine signalbildende Gruppe verfügen. Die Spaltung des Substrats führt zur Freisetzung der signalbildenden Gruppe. Die physikalischen oder chemischen Eigenschaften der freigesetzten signalbildenden Gruppe unterscheiden sich von den Eigenschaften der an das Peptid gekoppelten Gruppe und können mit Hilfe geeigneter Verfahren quantitativ bestimmt werden. Als signalbildende Gruppe eignen sich z. B. dem Fachmann bekannte Luminophore, Fluorophore oder Chromophore, die mittels optischer Verfahren, wie beispielsweise Lumineszenz-, Fluoreszenz- oder Absorptionsmessungen gemessen werden können. Da die Signalstärke mit der Menge an gespaltenem Substrat korreliert, kann so die Plasminogenaktivator-aktivierende Aktivität der FSAP bestimmt werden. Bevorzugterweise wird ein niedermolekulares Substrat aus der Gruppe S-2444™ (Glu-Gly-Arg-para-Nitroanilin; Chromogenix Instrumentation Laboratory S.p.A., Milano, Italien), Pefachrom® uPA (Ala-Gly-Arg-para-Nitroanilin [Pefa-5221]; Pentapharm Ltd., Basel, Schweiz) und Chromozym™ U (Roche Applied Science, Indianapolis, USA) verwendet.

Unter einer "Probe" ist im Sinne der Erfindung das Material zu verstehen, das die FSAP vermutlich enthält. Der Begriff "Probe" umfasst biologische Flüssigkeiten, insbesondere Körperflüssigkeiten von Menschen und Tieren, wie Blut, Plasma, Serum; menschliche oder tierische Ausscheidungen; Extrakte menschlicher oder tierischer Gewebe; Zellkulturüberstände oder -extrakte von Kulturen von isolierten menschlichen oder tierischen Zellen oder von Mikroorganismen, die die FSAP rekombinant exprimieren.

Erfindungsgemäß wird der Probe vor Zugabe des Plasminogenaktivators mindestens ein Aktivator der FSAP zugegeben. Bevorzugte Aktivatoren sind hochmolekulares Dextransulfat (Molekulargewicht zwischen etwa 40.000 und etwa 1.000.000 Da) aber auch niedermolekulares Dextransulfat (Molekulargewicht zwischen etwa 1.500 und etwa 40.000 Da) und deren Salze, besonders Natrium- und Calciumsalze, unfraktioniertes Heparin, Heparansulfat, Hyaluronsäure, Kaolin, RNA zum Beispiel Gesamt-RNA von Hefe (Saccharomyces cerevisiae) oder Poly-L-Lysin.

Die Probe wird mit dem FSAP-Aktivator bei einem pH-Wert von 4 bis 8, bevorzugt bei einem pH-Wert von 5 bis 7, besonders bevorzugt bei einem pH-Wert von 6,0 +/- 0,5 inkubiert. Der pH-Wert kann vor oder nach Zugabe des FSAP-Aktivators durch Zugabe eines geeigneten Puffers zur Probe eingestellt werden oder dadurch, dass der FSAP-Aktivator bereits in einem geeigneten Puffer gelöst ist.

Der resultierende Testansatz, umfassend die Probe mit dem FSAP-Aktivator und mit einem pH-Wert von 4 bis 8, wird erfindungsgemäß für einen Zeitraum von 30 Sekunden bis 1 Stunde, bevorzugt von 10 bis 30 Minuten, besonders bevorzugt von 15 Minuten von der Bereitstellung des Testansatzes bis zu dem Zeitpunkt inkubiert, in dem der Plasminogenaktivator dem Testansatz zugegeben wird.

Die Inkubation des Testansatzes vor Zugabe des Plasminogenaktivators erfolgt bevorzugt bei einer Temperatur zwischen etwa +4 und +50 °C, bevorzugt zwischen etwa +30 und +37 °C und besonders bevorzugt bei einer Temperatur von etwa +37 °C.

Ein weiterer Gegenstand der vorliegenden Erfindung ist ein diagnostisches Verfahren zur Erkennung von Personen mit genetisch bedingter hetero- oder homozygoter Expression der MRI-Variante der Faktor VII-aktivierenden Protease (FSAP), wobei die Plasminogenaktivator-aktivierende Aktivität der FSAP in einer Körperflüssigkeitsprobe eines Individuums mit dem erfindungsgemäßen homogenen Verfahren in Abwesenheit sowie in Anwesenheit eines differenziellen Aktivitätsmodulators bestimmt wird, welcher die spezifische Aktivierung der FSAP in Proben von Personen mit genetisch bedingter hetero- oder homozygoter Expression der MRI-Variante der FSAP in anderem Ausmaß ändert als in Proben von Personen, die die MRI-Variante der FSAP nicht exprimieren.

Ein analoges diagnostisches Verfahren, bei dem die Plasminogenaktivator-aktivierende Aktivität der FSAP allerdings in einem heterogenen Verfahren bestimmt wird, ist in EP 1801233 A1 beschrieben.

Der differenzielle Aktivitätsmodulator wird bevorzugterweise mit der zu untersuchenden Probe vermischt und inkubiert bevor der erfindungsgemäße Aktivator der FSAP mit der Probe vermischt wird.

Bevorzugte differenzielle Aktivitätsmodulatoren, die Plasminogenaktivator-aktivierende Aktivität der FSAP in Proben von Trägern der MR I-Variante der FSAP in anderem Ausmaß, d. h. signifikant stärker oder schwächer, modulieren als in Proben von Nichtträgern der MR I-Variante der FSAP (Wildtypen), sind beispielsweise Aprotinin oder monoklonale anti-FSAP-Antikörper, insbesondere ein monoklonaler Antikörper (MAK 1102-570), der von der Hybridomazelllinie DSM ACC2726 gebildet wird, welche bei der DSMZ - Deutsche Sammlung von Mikroorganismen und Zellkulturen GmbH, Inhoffenstraße 7 B, 38124 Braunschweig, Deutschland hinterlegt ist (siehe auch EP 1630175 A1). Dieser monoklonale Antikörper inhibiert die Aktivierung der FSAP in Proben von Trägern der MR I-Variante der FSAP stärker als in Wildtyp-Proben.

### FIGUR 1

Graphische Darstellung der Absorptionsmessungen (OD 405 nm) von 6 FSAP Wildtyp-Proben (WT) und 6 FSAP-MRI-Proben (MRI) in einem erfindungsgemäßen homogenen Test zur Bestimmung der FSAP-Aktivität (schraffierte Balken) und in einem heterogenen Test zur Bestimmung der FSAP-Aktivität gemäß dem Stand der Technik (unschraffierte Balken) (siehe auch Beispiel 1).

### FIGUR 2

Graphische Darstellung des Einflusses verschiedener Substanzen auf die Prourokinase-aktivierende Aktivität der FSAP im erfindungsgemäßen homogenen Verfahren (siehe auch Beispiel 2).

### BEISPIELE

### Beispiel 1: Erfindungsgemäße Bestimmung der Prourokinase-aktivierenden Aktivität der FSAP in humanen Plasmaproben von Wildtypen und Trägern der MRI-Variante der FSAP mit hochmolekularem Dextransulfat als FSAP-Aktivator

### Materialien

| | |
|---|---|
| Probenpuffer: | 20 mM Natriumcitrat |
| | 150 mM NaCl |
| | 100 mM L-Argininmonohydrochlorid |
| | 1 % BSA |
| | 0,1 % Tween^{®} 80 |
| | pH 6,0 |
| | |
| Testpuffer: | 50 mM Tris |
| | 150 mM NaCl |
| | 15 mM CaCl₂ |
| | 0,2 % Tween^{®} 80 |
| | pH 8,0 |

Jeweils 10 µL einer Citratplasmaprobe von jeweils 6 Wildtypen und 6 Trägern der MRI-Variante der FSAP wurden mit 20 µL Probenpuffer verdünnt und für 15 Minuten bei 37 °C in einer 300 µL Polystrol-Reaktionsküvette vorinkubiert. Die FSAP-Aktivierung erfolgte durch Zugabe von weiteren 20 µL Probenpuffer enthaltend 20 µg/mL hochmolekulares Dextransulfat (Na-Salz von Dextransulfat mit einem durchschnittlichen relativen Molekulargewicht (MG) von > 500.000 Da, Sigma-Aldrich Chemie GmbH, München, Deutschland) und anschließender Inkubation des Testansatzes für 15 Minuten bei 37 °C. Nach der 15minütigen Inkubation wurden dem Testansatz 150 µL Testpuffer enthaltend 2,5 µg/mL Prourokinase (scuPA, Landing Biotech, Inc., Newton, MA, USA) und 0,3 mM eines pNA-markierten Urokinase-Peptidsubstrats (S-2444™, Chromogenix, Mölndal, Schweden) zugegeben. Die Absorption des Testansatzes wurde bei einer Wellenlänge von 405 nm in einem Spektrophotometer (SpectraMax^{®} Plus³⁸⁴, Molecular Devices, Inc., CA, USA) über 30 Minuten bei 37 °C gemessen.

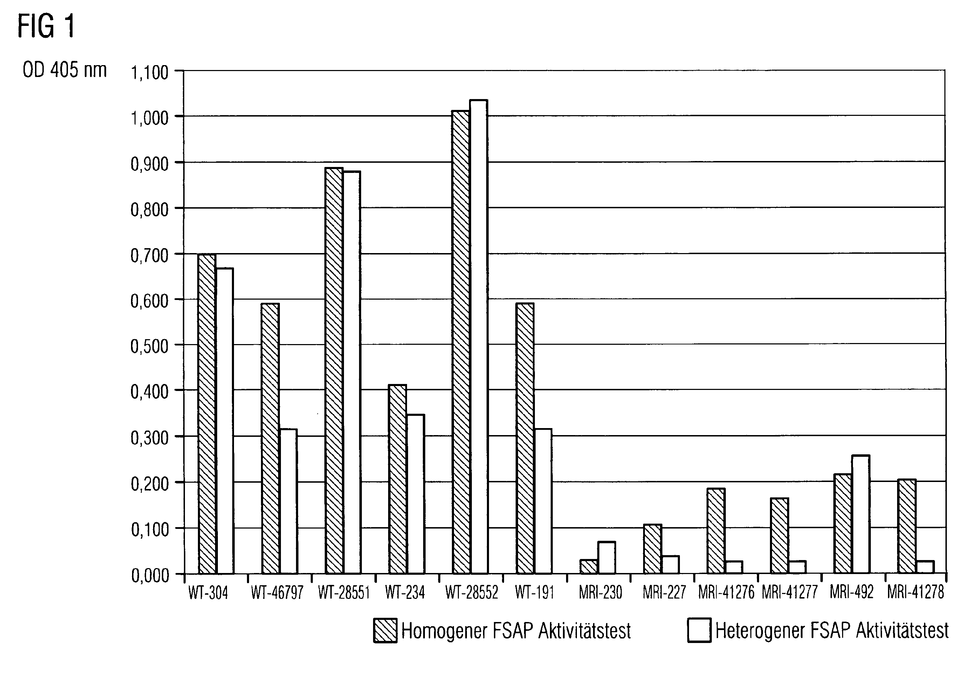

In Figur 1 sind die Absorptionsänderungen, die mit dem erfindungsgemäßen homogenen Verfahren in den diversen Patientenproben gemessen wurden, im Vergleich zu Absorptionsänderungen, die mit einem heterogenen Verfahren zur Bestimmung der Prourokinase-aktivierenden Aktivität der FSAP gemäß dem Stand der Technik gemessen wurden, dargestellt. In beiden Verfahren unterscheiden sich die Absorptionswerte (OD 405 nm) der FSAP Wildtyp-Proben (WT) deutlich von den Absorptionswerten der FSAP MRI-Proben (MRI). Das erfindungsgemäße homogene Verfahren erlaubt neben der Bestimmung der Prourokinase-aktivierenden Aktivität der FSAP in Patientenproben durch die Festlegung eines geeigneten Cutoffs auch eine Differenzierung zwischen Proben von Trägern der FSAP MRI-Variante und FSAP Wildtyp-Proben, analog zum heterogenen Verfahren.

### Beispiel 2: Verschiedene FSAP-Aktivatoren im erfindungsgemäßen homogenen Verfahren zur Bestimmung der FSAP-Aktivität

Mit dem Testaufbau, wie in Beispiel 1 beschrieben, wurden folgende Substanzen als potenzielle FSAP-Aktivatoren getestet: Protaminsulfat (600 µg/mL), Gesamt-RNA aus Saccharomyces cerevisiae (600 µg/mL), Kaolin (600 µg/mL), Poly-L-Lysin (5 µg/mL), hochmolekulares (HMW) Dextransulfat (MW > 500.000 Da; 5 µg/mL), niedermolekulares (LMW) Dextransulfat (MW = 6-10 kDa; 5 µg/mL), unfraktioniertes Heparin (1 IU/mL). Als Probe wurde humanes Normalcitratplasma verwendet. Als Kontrolle wurde ein Testansatz gemessen, dem kein potenzieller FSAP-Aktivator zugegeben wurde, sondern lediglich Probenpuffer.

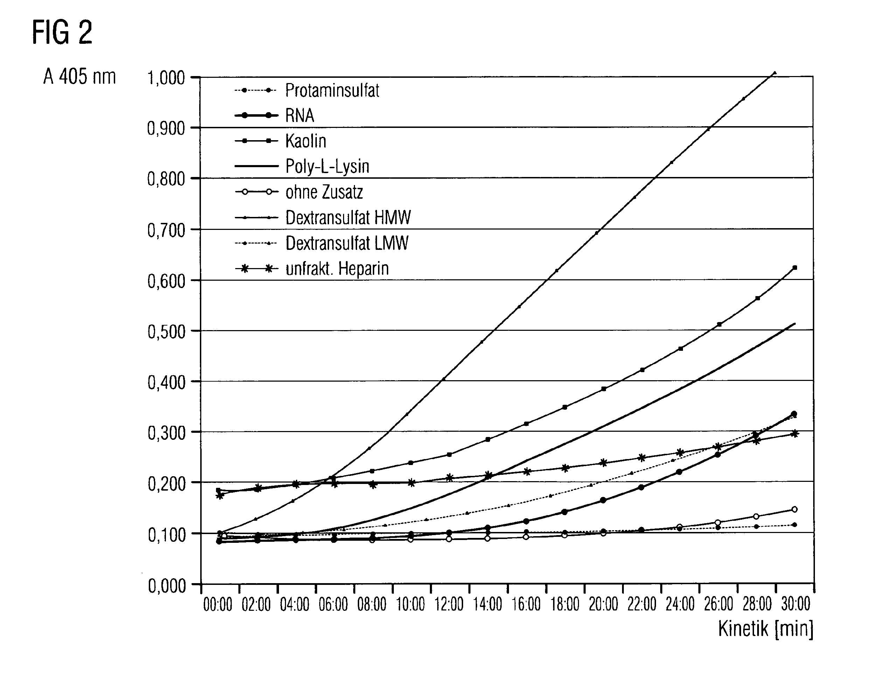

Die Absorptionsmessungen sind in Figur 2 dargestellt. Protaminsulfat eignet sich nicht als FSAP-Aktivator, da in diesem Testansatz die gemessene FSAP-Aktivität nicht höher ist als die Aktivität in dem Testansatz ohne weiteren Zusatz. Alle übrigen getesteten Substanzen bewirken eine Aktivierung der Prourokinase-aktivierenden Aktivität der FSAP und wirken daher als FSAP-Aktivatoren, die eine Bestimmung der FSAP-Aktivität in einem homogenen Verfahren überhaupt erst ermöglichen.

### Beispiel 3: Differenzielle Inhibition der Dextransulfat-Aktivierung der FSAP mit einem monoklonalen anti-FSAP-Antikörper

Mit dem homogenen Testaufbau, wie in Beispiel 1 beschrieben, wurde getestet, ob sich die FSAP-Aktivierung durch hochmolekulares Dextransulfat (durchschnittliches MW von 500.000 g/mol) in FSAP WT-Proben in anderem Ausmaß modulieren lässt als in Proben von FSAP MRI-Trägern. Dazu wurden jeweils 10 µL Citratplasmaprobe mit 20 µL Probenpuffer enthaltend einen monoklonalen anti-FSAP-Antikörper (1,25 - 5 µg/mL), der von der Hybridomazelllinie DSM ACC2726 gebildet wird, verdünnt. Das Gemisch wurde für 15 Minuten bei 37 °C inkubiert, bevor die Dextransulfatlösung zur Aktivierung der FSAP zugegeben wurde.

Die Testergebnisse sind in Tabelle 1 zusammengefasst. Tabelle 1 zeigt die Prourokinase-aktivierende Aktivität der FSAP von Trägern und Nichtträgern der FSAP MR I-Variante ohne und mit Zusatz verschiedener Konzentrationen des monoklonalen anti-FSAP Antikörpers MAK 1102-570 (MAK570). Die FSAP-Aktivität in Proben von Trägern der MRI-Variante der FSAP wird bei Zusatz von Anti-FSAP MAK 1102-570 in einer Konzentration von 2,5 µg/mL bis 5 µg/mL, signifikant stärker inhibiert als in Proben von Nichtträgern (WT), wobei eine Differenzierung zwischen Trägern und Nichtträgern der FSAP MRI-Variante (Ratio WT/MRI) deutlich gegenüber dem Ansatz ohne Antikörper-Zusatz verbessert wird. Der "Ratio WT/MRI" wurde aus den Mittelwerten der WT Proben und MRI Proben berechnet. Die Inhibierbarkeit der Prourokinase-aktivierenden Aktivität der FSAP durch den FSAP-spezifischen Antikörper (MAK570) zeigt die überwiegend FSAP-spezifische Aktivierung der Prourokinase im erfindungsgemäßen homogenen Verfahren.

**Tabelle 1**

| | Δ OD 405 nm (30 Minuten Substratinkubation) | | | | | | |
|---|---|---|---|---|---|---|---|
| | 67µg/mL | 34µg/mL | 17µg/mL | 10µg/mL | 5µg/mL | 2,5µg/mL | ohne |
| **Proben ID** | MAK570 | MAK570 | MAK570 | MAK570 | MAK570 | MAK570 | MAK |
| 4434 (WT) | 0,139 | 0,185 | 0,17 | 0,151 | 0,27 | 0,813 | 0,735 |
| 4404 (WT) | 0,066 | 0,06 | 0,044 | 0,039 | 0,031 | 0,612 | 0,654 |
| 4427 (WT) | 0,128 | 0,067 | 0,055 | 0,054 | 0,411 | 0,935 | 0,855 |
| 4424 (WT) | 0,103 | 0,049 | 0,045 | 0,035 | 0,12 | 0,824 | 0,762 |
| | | | | | | | |
| 4364 (MRI) | 0,154 | 0,005 | 0,193 | 0,041 | 0,097 | 0,087 | 0,457 |
| 4377 (MRI) | 0,037 | 0,037 | 0,032 | 0,021 | 0,029 | 0,039 | 0,454 |
| 4363 (MRI) | 0,132 | 0,115 | 0,158 | 0,093 | 0,048 | 0,086 | 0,067 |
| 4433 (MRI) | 0,079 | 0,093 | 0,048 | 0,056 | 0,035 | 0,051 | 0,839 |
| | | | | | | | |
| **Ratio WT/MRI** | **0,95** | **0,97** | **0,49** | **1,32** | **3,98** | **12,11** | **1,65** |

## Patentansprüche

1. Homogenes Verfahren zur Bestimmung der Plasminogenaktivator-aktivierenden Aktivität der Faktor VII-aktivierenden Protease (FSAP) in einer Probe, wobei die Probe mit einem Plasminogenaktivator und einem Plasminogenaktivator-spezifischen Substrat vermischt wird und die Plasminogenaktivatoraktivität gemessen wird, **dadurch gekennzeichnet, dass** der Probe vor der Zugabe des Plasminogenaktivators mindestens ein Aktivator der FSAP-Aktivität zugegeben wird und der pH-Wert der Probe auf einen Wert von 4 bis 8 eingestellt wird.

2. Verfahren nach Anspruch 1, wobei der Probe vor der Zugabe des Plasminogenaktivators mindestens ein Aktivator der FSAP-Aktivität aus der Gruppe hochmolekulares Dextransulfat, niedermolekulares Dextransulfat, unfraktioniertes Heparin, Heparansulfat, Hyaluronsäure, Kaolin, RNA und Poly-L-Lysin zugegeben wird.

3. Verfahren nach einem der vorherigen Ansprüche, wobei der pH-Wert der Probe auf einen Wert von 5 bis 7, besonders bevorzugt auf einen Wert von 6,0 +/- 0,5 eingestellt wird.

4. Verfahren nach einem der vorherigen Ansprüche, wobei die Probe nach der Zugabe des Aktivators der FSAP-Aktivität und nach der Einstellung des pH-Wertes für einen Zeitraum von mindestens 30 Sekunden bis 1 Stunde, bevorzugt 10 bis 30 Minuten, insbesondere bevorzugt 15 Minuten inkubiert wird, bevor der Plasminogenaktivator zugegeben wird.

5. Verfahren nach Anspruch 4, wobei die Inkubation bei einer Temperatur zwischen einschließlich +4 bis +50 °C, bevorzugt zwischen einschließlich +30 und +37 °C und besonders bevorzugt bei +37 °C erfolgt.

6. Diagnostisches Verfahren zur Erkennung von Personen mit genetisch bedingter hetero- oder homozygoter Expression der MRI-Variante der Faktor VII-aktivierenden Protease (FSAP), bei dem die Plasminogenaktivator-aktivierende Aktivität der FSAP, die in einer Probe, in Abwesenheit sowie in Anwesenheit eines differenziellen Aktivitätsmodulators bestimmt wird, welcher die spezifische Aktivierung der FSAP in Proben von Personen mit genetisch bedingter hetero- oder homozygoter Expression der MRI-Variante der FSAP in anderem Ausmaß ändert als in Proben von Personen, die die MR I-Variante der FSAP nicht exprimieren, **dadurch gekennzeichnet, dass** die Plasminogenaktivator-aktivierende Aktivität der FSAP mit einem homogenen Verfahren gemäß einem der Ansprüche 1 bis 5 bestimmt wird.

7. Verfahren gemäß Anspruch 6, wobei der differenzielle Aktivitätsmodulator die Plasminogenaktivator-aktivierende Aktivität der FSAP in Proben von Personen mit genetisch bedingter hetero- oder homozygoter Expression der MRI-Variante der FSAP, in stärkerem Ausmaß inhibiert oder aktiviert werden, als in Proben von Personen, die die MRI-Variante der FSAP nicht exprimieren.

8. Verfahren gemäß Anspruch 7, wobei der differenzielle Aktivitätsmodulator, der die FSAP-Aktivierung in Proben von Personen, die die MRI-Variante der FSAP exprimieren, in stärkerem Ausmaß inhibiert oder aktiviert, ein monoklonaler anti-FSAP-Antikörper ist.
